(19) Europäisches Patentamt
European Patent Office
Office européen des brevets

(11) Publication number: **0 060 079**
**B1**

(12) # EUROPEAN PATENT SPECIFICATION

(45) Date of publication of patent specification: **21.11.85**

(21) Application number: **82301065.7**

(22) Date of filing: **03.03.82**

(51) Int. Cl.$^4$: **C 11 D 1/12,** C 11 D 1/29, B 01 F 17/00, C 07 C 143/46, E 21 B 43/20

(54) **Surfactant compounds, their preparation and use.**

(30) Priority: **07.03.81 GB 8107232**
**12.03.81 GB 8107815**

(43) Date of publication of application:
**15.09.82 Bulletin 82/37**

(45) Publication of the grant of the patent:
**21.11.85 Bulletin 85/47**

(84) Designated Contracting States:
**BE DE FR GB IT NL**

(56) References cited:
**DE-A-2 241 567**
**DE-A-2 558 548**
**DE-A-2 642 276**

**The file contains technical information submitted after the application was filed and not included in this specification**

(73) Proprietor: **The British Petroleum Company p.l.c.**
**Britannic House Moor Lane**
**London EC2Y 9BU (GB)**

(72) Inventor: **Callaghan, Ian Charles The British Petroleum Company p.l.c. Chertsey Road Sunbury-on-Thames Middlesex, TW16 7LN (GB)**
Inventor: **Clint, John Howard The British Petroleum Company p.l.c. Chertsey Road Sunbury-on-Thames Middlesex, TW16 7LN (GB)**
Inventor: **Kidd, David Arthur The British Petroleum Company p.l.c. Chertsey Road Sunbury-on-Thames Middlesex, TW16 7LN (GB)**

(74) Representative: **Crack, Richard David et al**
**c/o The British Petroleum Company plc Patents Division Chertsey Road**
**Sunbury-on-Thames Middlesex, TW16 7LN (GB)**

**EP 0 060 079 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new class of compounds suitable for use as surfactants and to a method for the recovery of oil from a reservoir utilising said surfactants.

DE—A 2,558,548 discloses a method for the recovery of oil from a reservoir which utilises a waterflood containing a surfactant of general formula:

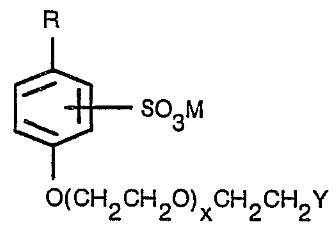

wherein R represents an alkyl group containing 6—18 carbon atoms, M is a metallic cation or an ammonium ion, x is a number from 1—15 and Y is an OH group or a $SO_3M$ group.

In these compounds the aryl group is directly connected to the alkyl group R.

We have now discovered that if an alkoxy bridge is interposed between the alkyl and aryl groups, the resulting compounds have a low tendency to adsorb on inorganic surfaces such as reservoir rock.

Thus according to the present invention there is provided an anionic surfactant containing alkyl, alkoxy, aromatic and sulphonate groups wherein the aryl group is connected to the alkyl group by means of an alkoxy bridge and the surfactant is of general formula:

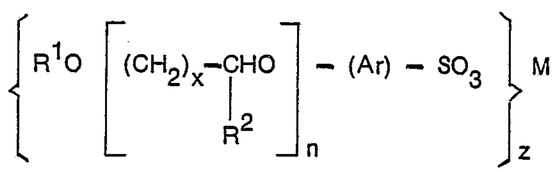

where $R^1$ is a branched or straight chain hydrocarbon radical having from 8 to 20 carbon atoms; x is either 1 or 2; $R^2$ is a hydrogen atom or a $CH_3$ radical; n is an integer from 1 to 20 inclusive; M is ammonium, an alkaline earth metal or an alkali metal; z is 1 or 2 and Ar is a benzene or naphthalene nucleus which is optionally substituted by a group of formula:

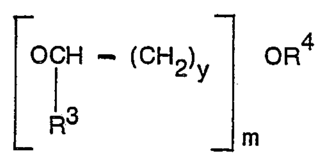

where $R^4$ is as defined for $R^1$; $R^3$ is as defined for $R^2$; m is as defined for n and y is as defined for x, or by one or more substituents such as a $C_1$ to $C_3$ alkyl radical.

In the present specification, integers which indicate specific numbers of repeating units refer to a mean or average.

According to another aspect of the present invention there is provided a method for the recovery of oil from a reservoir which method comprises injecting into the reservoir an aqueous surfactant composition containing a surfactant as hereinbefore described through a first well and recovering oil from a second well.

The composition may contain in addition a non-ionic surfactant.

The invention is illustrated by the following Examples.

EXAMPLE 1

Preparation of

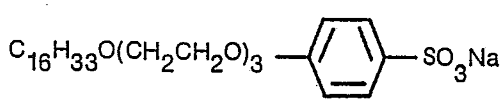

(III)

The preparation is a two stage reaction from readily available commercial intermediates:

1. $C_{16}H_{33}O(CH_2CH_2O)_3H$ (I) + $PBr_3$ $\xrightarrow{CaCO_3}$ $C_{16}H_{33}O(CH_2CH_2O)_2CH_2CH_2Br$ (II)

2. 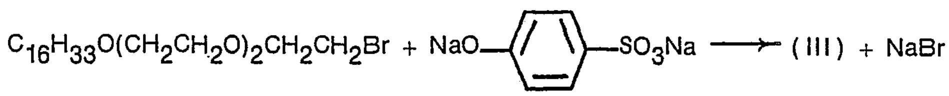

Stage 1

| | | |
|---|---|---|
| (I) | $C_{16}H_{33}O(CH_2CH_2O)_3H$ | 50 gm equivalent to 0.133 mole |
| | $PBr_3$ | 12 gm (4.2 ml) equivalent to 0.045 mole |
| | $CaCO_3$ | 13.3 gm |
| | Xylene | 300 ml + 20 ml |

The 50 gm of (I). 133 gm of calcium carbonate and 300 ml of xylene were charged to a 1 litre flask fitted with thermometer, mechanical stirrer, dropping funnel and calcium chloride guard tube. The phosphorus tribromide in 20 ml of xylene was run into the stirred slurry at a rate to not exceed a temperature of 40°C (the reaction is exothermic: time of addition about 20 minutes). The mixture was stirred for a further two hours at ambient temperature and then diluted with 100 ml of xylene, filtered through magnesium sulphate and the residue after the xylene was evaporated which consisted of compound (II) removed and weighed.

Stage 2

| | |
|---|---|
| Compound (II) | 41 gm equivalent to 0.094 mole |
| $NaOC_6H_4SO_3NA\ 3H_2O$ | 30 gm equivalent to 0.094 mole |
| Diethylene glycol | 150 ml |

The sodium phenol sulphonate and diethylene glycol were charged to a 500 ml flask fitted with a thermometer, reflux condenser and mechanical stirrer and warmed to 70—80°C to dissolve the phenol sulphonate. The 41 gm of compound (II) previously prepared were added and stirred at reflux for 3 hours, the temperature being 155°C. The solution became opaque and solid began to crystallise out after about 1 hour. The mixture was allowed to cool, diluted with ethanol and filtered. The residue was dried at 90°C/200 mm. The residue was recrystallised from ethanol after filtering hot to remove sodium bromide and the product dried at 90°C/200 mm.

Analysis of the product gave the following

| | C | H | O | S | Na | Br | $H_2O$ |
|---|---|---|---|---|---|---|---|
| Found% | 61 | 8.9 | 19.3 | 6.8 | 4.0 | 0.03 | 0.2 |
| Theoretical% | 60.8 | 8.9 | 20.3 | 5.8 | 4.2 | | |

Evidence of surfactant properties was as follows: at 25°C the critical micelle concentration was $2 \times 10^{-5}$ molar. The surface tension of a $2 \times 10^{-5}$ molar solution of the compound III at 25°C is 43 $mNm^{-1}$ (compared with pure water of 72 $mNm^{-1}$).

## EXAMPLE 2

Preparation of

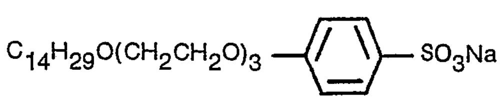

(IV)

The preparation was exactly as described in Example 1 except that the starting material was

$C_{14}H_{29}O(CH_2CH_2O)_3H$

instead of compound (I).

Analysis of the recrystallised product gave

| | C | H | O | S | Na | Br | $H_2O$ |
|---|---|---|---|---|---|---|---|
| Found% | 59.5 | 8.5 | 21.0 | 6.2 | 4.8 | 0.05 | 0.25 |
| Theoretical% | 59.5 | 8.6 | 21.3 | 6.1 | 4.4 | 0 | |

The oxygen was determined by difference in both Examples.

The compounds prepared in the Examples have the advantage of low adsorption on inorganic surfaces such as reservoir rock.

## EXAMPLE 3

Preparation of

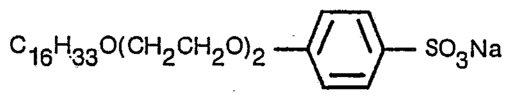

The preparation was carried out exactly as described in Example 1 except that the starting material was $C_{16}H_{33}O(CH_2CH_2O)_2H$. Analysis of the recrystallised product gave

| | C | H | S | Na | Br | $H_2O$ |
|---|---|---|---|---|---|---|
| Found% | 59.5 | 9.2 | 5.8 | 3.9 | 0.7 | 0.6 |
| Theoretical% | 61.4 | 8.9 | 6.3 | 4.5 | — | — |

## EXAMPLE 4

Preparation of

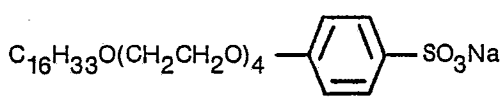

The preparation was carried out exactly as described in Example 1 except that the starting material was

$C_{16}H_{33}O(CH_2CH_2O)_4H$

Analysis of the recrystallised product gave

| | C | H | S | Na | Br | $H_2O$ |
|---|---|---|---|---|---|---|
| Found% wt | 59.2 | 9.3 | 6.4 | 3.3 | 0.2 | 0.5 |
| Theoretical wt | 60.4 | 9.0 | 5.4 | 3.8 | — | — |

A 0.2% solution was prepared in Forties sea water. The interfacial tension against stabilised Forties crude oil at 70°C was $5.15 \times 10^{-2} mNm^{-1}$.

## EXAMPLE 5

Preparation of

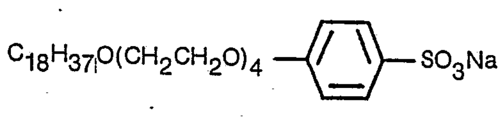

The preparation was carried out exactly as described in Example 1 except that the starting material was

$C_{18}H_{37}O(CH_2CH_2O)_4H$

Analysis of the recrystallised product gave

| | C | H | S | Na | Br | $H_2O$ |
|---|---|---|---|---|---|---|
| Found% wt | 61.6 | 9.5 | 6.2 | 2.4 | 0.04 | 0.2 |
| Theoretical% wt | 61.5 | 9.2 | 5.1 | 3.7 | — | — |

A 0.4% solution was prepared in Forties sea water. The interfacial tension against stabilised Forties crude oil at 70°C was $7.29 \times 10^{-3}mNm^{-1}$.

## EXAMPLE 6

Preparation of

1-(3,6,9,12-tetraoxaoctacosanoxy)-naphthalene sulphonic acid, sodium salt

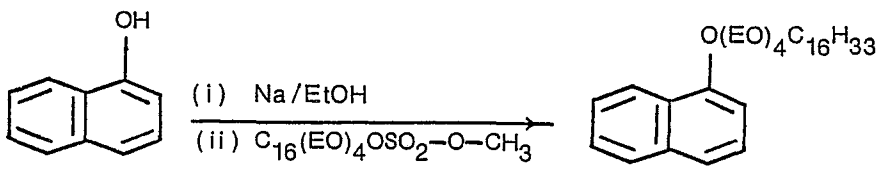


1

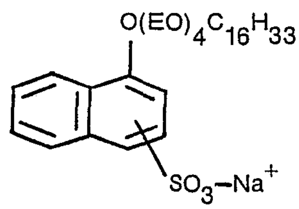

2

1-Naphthol (12.6 g, 0.09 mol) was added to a solution of sodium ethoxide (ex 2.0 g Na) in ethanol (300 $cm^3$) and stirred at room temperature for 15 minutes. To this was slowly added a 1:1 solution of the mono tosyl ester of 3,6,9,12-tetraoxaoctacosanol (47.0 g, 0.08 mol) in ethanol, followed by boiling for 30 minutes. Removal of ethanol left a semi-solid residue which was partitioned between $H_2O/CHCl_3$. The organic phase was washed with 1M NaOH (×3), water (×3) and dried ($MgSO_4$). Removal of $CHCl_3$ gave 45.7 g of a purple liquid, identified (MS) as *1*.

To a solution of *1* (20 g 0.04 mol) in chloroform maintained at 0° was added with vigorous stirring chlorosulphonic acid (4.4 g, 0.038 mol). After 30 minutes, chloroform was stripped off and the residue dissolved in 1:1 $H_2O$/acetone. The pH of this solution was adjusted to 7 with $NaHCO_3$ and solvent allowed to evaporate. Addition of a large amount of acetone gave a mixture of compounds *2* containing mainly compounds in which the sulphonate was attached to the 4 position as a colourless solid (16.8 g, 0.027 mol), which recrystallised as platelets from aq.acetone.

NMR spectra ($^1H$&$^{13}C$) were compatible with structure 2 above.

| | C | H | S | Na |
|---|---|---|---|---|
| Found% wt | 62.1 | 8.9 | 4.8 | 3.0 |
| Theoretical% wt | 63.1 | 8.5 | 5.0 | 3.4 |

A 0.2% solution of the product in Forties sea water had an interfacial tension against stabilised Forties crude oil of $8 \times 10^{-3}mNm^{-1}$ at 70°C.

EXAMPLE 7

Preparation of

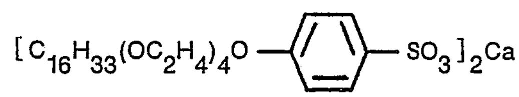

$C_{16}H_{33}(OC_2H_4)_4OH$

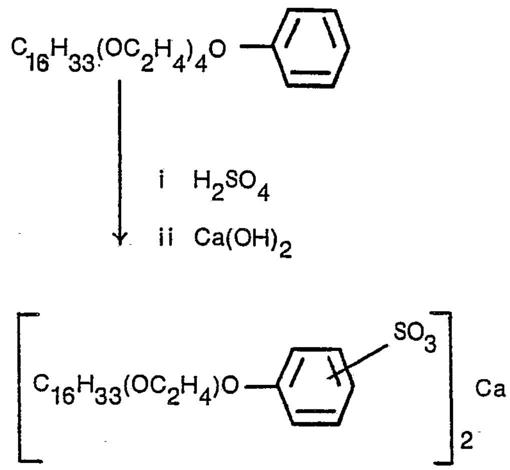


(a) $C_{16}H_{33}O(CH_2CH_2O)_4H$ (0.1 mole) was dissolved in dry xylene and sodium hydride (80% dispersion in oil) (0.1 mole) added in portions. The reaction mixture was heated to 100°C and 2-bromoethoxybenzene (0.1 mole) was run in with stirring, then the mixture was stirred at 139—140°C for 5 hours. Sodium bromide was then filtered off and the product absorbed on chromatographic alumina. Elution with 1:1 ethyl acetate/heptane affords

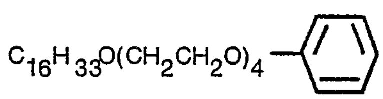

in 50% yield.

(b) 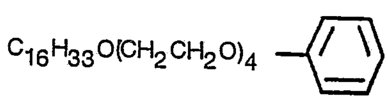 (35.3m mole)

was dissolved in carbon tetrachloride and concentrated sulphuric acid stirred in over 5 minutes. The reaction was exothermic (25—37°C). After standing overnight the mixture was evaporated, dissolved in water and neutralised with $Ca(OH)_2$.

The product was absorbed on silica and eluted with isopropanol/water (4:1). The sulphonate was obtained pure after recrystallising from ethanol/ether.

| | C | H | S | Ca | Br | $H_2O$ |
|---|---|---|---|---|---|---|
| Found% wt | 59.8 | 9.1 | 6.0 | 3.2 | 0.2 | 0.2 |
| Theoretical% wt | 60.3 | 9.0 | 5.0 | 3.1 | — | — |

The compound was dissolved in Forties sea water to give a 2000 ppm solution. The interfacial tension against stabilised Forties crude oil was $6.42 \times 10^{-2}$mNm$^{-1}$.

Example 8

A laboratory sand column composed of sand extracted from the Forties Field in the North Sea was saturated with filtered sea water followed by degassed Forties crude oil and then subjected to a complete sea water flood by passing filtered sea water.

Then a surfactant flood consisting of 3000 ppm solution of the compound prepared in Example 1 in

filtered sea water at 70°C was passed through the column. A well defined oil bank was formed and when between 1.3 and 1.9 pore volumes of the surfactant flood had been passed into the column, the oil bank containing 24% of the residual crude oil remaining after the sea water flood was liberated from the column.

One advantage of the above described surfactant is that it can be used alone in the sea water or formation water. This reduces the chromatographic separation which takes place in the reservoir. A further advantage of this surfactant is that adsorption from solution in sea water onto reservoir sand is very low, about 0.15 mg surfactant per g of sand. This reduces adsorptive losses which would take place in the reservoir.

Example 9

The sand column test described in Example 8 was repeated under identical conditions with the exception that a solution of a 1:1 mole mixture of the compound prepared in Example 1 and nonylphenolethoxylate (which contained 6 molecules of ethylene oxide per molecule of nonyl phenol) at a concentration of 2000 ppm was employed. The per cent residual oil released was 53% and the production range 2 to 8 pore volumes.

Examples 10 and 11 are recorded in the following table:

| Surfactant Mixture in Sea Water | Concentration ppm | % Residual Oil Released | Production Range (pore volume) |
|---|---|---|---|
| | Example 10 | | |
| Compound of Example 1 + nonyl phenol ethoxylate (9 moles of ethylene oxide) 1:1 mole | 2000 | 27 | 4 to 7 |
| | Example 11 | | |
| Compound of Example 1 + hexadecanol ethoxylated with 10 moles of ethylene oxide. 1:1 mole | 2000 | 42 | 2 to 7 |

The abbreviation ppm means parts per million by weight. The concentrations in Examples 9 to 11 refer to that of the sulphonate non-ionic surfactant mixture.

In addition to the above uses, the compounds of the present invention can be used as non-built detergents.

**Claims**

1. An anionic surfactant containing alkyl, alkoxy, aromatic and sulphonate groups characterised in that the aryl group is connected to the alkyl group by means of an alkoxy bridge and the surfactant is of general formula:

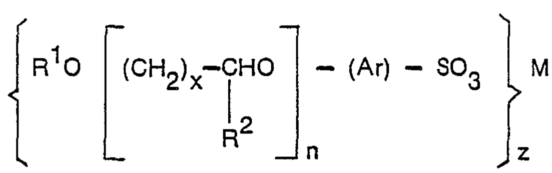

where $R^1$ is a branched or straight chain hydrocarbon radical having from 8 to 20 carbon atoms; x is either 1 or 2; $R^2$ is a hydrogen atom of a $CH_3$ radical; n is an integer from 1 to 20 inclusive; M is ammonium, an alkaline earth metal or an alkali metal; z is 1 or 2 and Ar is a benzene or naphthalene nucleus which is optionally substituted by a group of formula:

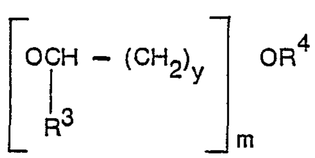

where $R^4$ is as defined for $R^1$; $R^3$ is as defined for $R^2$; m is as defined for n and y is as defined for x, or by one or more substituents such as a $C_1$ to $C_3$ alkyl radical.

2. An anionic surfactant according to claim 1 wherein Ar is a benzene nucleus characterised in that the oxyalkylene and sulphonate groups are disposed in 1:4 position on the benzene nucleus.

3. An anionic surfactant according to claim 1 wherein Ar is a naphthalene nucleus characterised in that the oxyalkylene and sulphonate groups are disposed in 1:4 positions on the naphthalene nucleus.

4. A method for the recovery of oil from a reservoir by injecting an aqueous surfactant composition into one well and recovering oil from a second well characterised in that the surfactant is an anionic surfactant according to any of the preceding claims.

5. A method for the recovery of oil from a reservoir according to claim 4 characterised in that the composition contains in addition a non-ionic surfactant.

**Revendications**

1. Tensio-actif anionique contenant des groupes alkyle, alcoxyle, aromatiques et sulfonate, caractérisé en ce que le groupe aryle est relié au groupe alkyle au moyen d'un pont alcoxyle et que le tensio-actif est de formule générale:

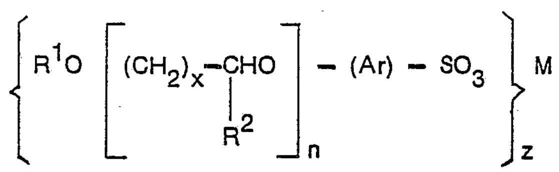

dans laquelle $R^1$ est un radical hydrocarbure à chaîne ramifiée ou droite ayant de 8 à 20 atomes de carbone; *x* est soit 1 soit 2; $R^2$ est un atome d'hydrogène ou un radical $CH_3$; *n* est un nombre entier de 1 à 20 inclusivement; M est un ion ammonium, un métal alcalino-terreux ou un métal alcalin; *z* est 1 ou 2 et Ar est un noyau benzène ou naphtalène qui est facultativement substitué par un groupe de formule:

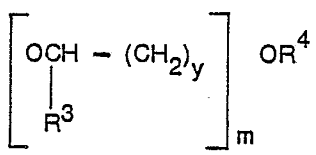

dans laquelle $R^4$ est tel que défini pour $R^1$; $R^3$ est tel que défini pour $R^2$; *m* est tel que défini pour *n* et *y* est tel que défini pour *x*, ou par un ou plusieurs substituants tels qu'un radical alkyle en $C_1$ à $C_3$.

2. Tensio-actif anionique selon la revendication 1, dans lequel Ar est un noyau benzène, caractérisé en ce que les groupes oxyalkylène et sulfonate sont disposés en positions 1:4 sur le noyau benzène.

3. Tensio-actif anionique selon la revendication 1, dans lequel Ar est un noyau naphtalène, caractérisé en ce que les groupes oxyalkylène et sulfonate sont disposés en positions 1:4 sur le noyau naphtalène.

4. Procédé pour la récupération de pétrole d'une réservoir par injection d'une composition aqueuse de tensio-actif dans un puits et récupération de pétrole d'un deuxième puits, caractérisé en ce que le tensio-actif est un tensio-actif anionique selon l'une quelconque des revendications précédentes.

5. Procédé pour la récupération de pétrole d'un réservoir selon la revendication 4, caractérisé en ce que la composition contient en outre un tensio-actif non ionique.

**Patentansprüche**

1. Anionisches oberflächenaktives Mittel, enthaltend Alkyl-, Alkoxy-, aromatische und Sulfonat-Gruppen, dadurch gekennzeichnet, daß die Aryl-Gruppe mit der Alkyl-Gruppe über eine Alkoxy-Brücke verbunden ist und das oberflächenaktive Mittel die allgemeine Formel

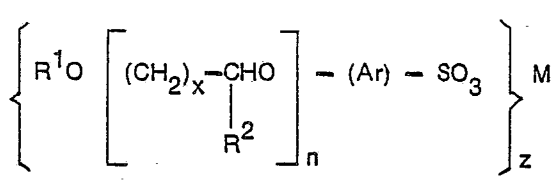

besitzt, in der

$R^1$ ein kettenverzweigter oder geradkettiger Kohlenwasserstoff-Rest mit 8 bis 20 Kohlenstoff-Atomen ist,

X entweder 1 oder 2 ist,

$R^2$ ein Wasserstoff-Atom oder ein $CH_3$-Rest ist,

n eine ganze Zahl von 1 bis 20 einschließlich ist,

M Ammonium, ein Erdalkali-Metall oder ein Alkali-Metall ist,
z 1 oder 2 ist und
Ar ein Benzol- oder Naphthalin-Kern ist, der gegebenenfalls durch eine Gruppe der Formel

$$\left[\begin{array}{l} OCH - (CH_2)_y \\ \;\;| \\ \;\;R^3 \end{array}\right]_m OR^4$$

in der
$R^4$ die für $R^1$ angegebenen Bedeutungen hat,
$R^3$ die für $R^2$ angegebenen Bedeutungen hat,
m die für n angegebenen Bedeutungen hat und
y die für x angegebenen Bedeutungen hat,
oder durch eine oder mehrere Substituenten wie ein $C_1$- bis $C_3$-Alkyl-Rest substituiert ist.

2. Anionisches oberflächenaktives Mittel nach Anspruch 1, dadurch gekennzeichnet, daß Ar ein Benzol-Kern ist und die Oxyalkylen- und Sulfonat-Gruppen sich in den 1,4-Stellungen des Benzol-Kerns befinden.

3. Anionisches oberflächenaktives Mittel nach Anspruch 1, dadurch gekennzeichnet, daß Ar ein Naphthalin-Kern ist und die Oxyalkylen- und Sulfonat-Gruppen sich in den 1,4-Stellungen des Naphthalin-Kerns befinden.

4. Verfahren zur Gewinnung on Öl aus einer Lagerstätte durch Einspritzen einer wäßrigen, oberflächenaktiven Zusammensetzung in ein Bohrloch und Gewinnen des Öls aus einem zweiten Bohrloch, dadurch gekennzeichnet, daß das oberflächenaktive Mittel ein anionisches oberflächenaktives Mittel nach irgendeinem der vorhergehenden Ansprüche ist.

5. Verfahren zur Gewinnung vo Öl aus einer Lagerstätte nach Anspruch 4, dadurch gekennzeichnet, daß die Zusammensetzung zusätzlich ein nicht-ionisches oberflächenaktives Mittel enthält.